Europäisches Patentamt

**European Patent Office**

**Office européen des brevets**

(11) Veröffentlichungsnummer: **0 075 904**
**B1**

(12) **EUROPÄISCHE PATENTSCHRIFT**

(45) Veröffentlichungstag der Patentschrift:
19.06.85

(51) Int. Cl.⁴: **C 12 N 5/00**

(21) Anmeldenummer: **82108880.4**

(22) Anmeldetag: **25.09.82**

(54) Verfahren zur Züchtung von humanen Fibroblasten und fibroblastenartigen Zellen.

(30) Priorität: **29.09.81 DE 3138597**

(43) Veröffentlichungstag der Anmeldung:
**06.04.83 Patentblatt 83/14**

(45) Bekanntmachung des Hinweises auf die Patenterteilung:
**19.06.85 Patentblatt 85/25**

(84) Benannte Vertragsstaaten:
**AT BE CH DE FR GB IT LI LU NL SE**

(73) Patentinhaber: **Boehringer Mannheim GmbH,
Sandhoferstrasse 116, D-6800 Mannheim 31 (DE)**

(72) Erfinder: **Northoff, Gerd-Heinrich, Dr. med., Am
Iller-Kanal 18/15, D-7910 Neu-Ulm (DE)**

(56) Entgegenhaltungen:
**CHEMICAL ABSTRACTS, Band 95, Nr. 13, 28. September
1981, Seite 511, Nr. 113272v, Columbus, Ohio, USA M.
JALKANEN: "Connective tissue activating
macromolecules in macrophage culture medium"
CHEMICAL ABSTRACTS, Band 88, Nr. 7, 13. Februar
1978, Seite 389, Nr. 48871d, Columbus, Ohio, USA B.
WENZEL et al.: "Differentiation between various effects
of cytotoxic fractions of lymphocyte culture medium"
CHEMICAL ABSTRACTS, Band 91, Nr. 7, 13. August
1979, Seite 482, Nr. 54211s, Columbus, Ohio, USA
OHNISHI SABURO et al.: "Lymphocyte-mediated
enhancement of collagen production by fibroblasts. I.
L929 fibroblasts as target cells"**

## Beschreibung

Die Erfindung betrifft ein neues Verfahren zur Züchtung von humanen Fibroblasten und fibroblastenartigen Zellen, insbesondere von Fruchtwasserzellen.

Fibroblasten sind Zellen im Bindegewebe, welche die Vorstufe der Fibrozyten darstellen. Die Züchtung dieser Zellen ist insbesondere deswegen von Bedeutung, da sie als Nährboden für die Vermehrung von Viren sowie zur Produktion und Isolierung biochemischer Substanzen, z. B. von Fibronectin, verwendet werden.

Zu den fibroblastenartigen Zellen sind die Fruchtwasserzellen zu zählen, da sie in ihrer Morphologie und in ihrem Wachstumsverhalten den eigentlichen Fibroblasten ähnlich sind. Fruchtwasserzellkulturen werden routinemäßig zur Frühdiagnose von Störungen im Chromosomensystem und im Stoffwechsel benutzt. Oft wird anhand dieser diagnostischen Untersuchung über die Notwendigkeit eines Schwangerschaftsabbruches entschieden. Um bei der Chromosomen-Diagnose ein brauchbares Ergebnis zu erhalten, ist es erforderlich, die Bestimmung an einer hinreichend großen Anzahl von Chromosomensätzen (Karyotopen) vorzunehmen. Die unterste Meßgrenze liegt bei 12—16 Karyotypen, das entspricht derselben Anzahl von Fruchtwasserzellen im Mitosestadium. Um Stoffwechselstörungen feststellen zu können, ist eine hinreichend große Biomasse erforderlich.

Bisher werden humane Fibroblasten und fibroblastenartige Zellen in handelsüblichen Kulturmedien gezüchtet, denen gegebenenfalls verschiedene Zusatzstoffe, wie z. B. Fibronectin, Dexomethason, Insulin, fetales Kälberserum u. ä., beigemischt werden. Übliche Kulturmedien sind im allgemeinen wäßrige Lösungen, die als wesentliche Bestandteile anorganische Salze, Vitamine und Aminosäuren enthalten. Beispiele für solche handelsüblichen Kulturmedien sind: das Kulturmedium HAM's F 10 der Firma SEROMED sowie das Kulturmedium RPMI 1640 der Firma Boehringer Mannheim GmbH. Zur Züchtung von Fruchtwasserzellkulturen wird häufig das Kulturmedium RPMI 1640 verwendet, dem 5—20% fetales Kälberserum zugesetzt wird (siehe hierzu Nadler, H. L. u. a., Amniotic Cell Culture in Human Chromosome Methodology, Yunis, J. J., Hrsg., Academic Press New York, 1974, S. 185—194). Gewöhnlich sind in dem Fruchtwasser jedoch nur wenige lebensfähige Zellen enthalten, so daß die Kultivierung der Fruchtwasserzellen oft mühsam und zeitaufwendig, zuweilen sogar völlig erfolglos ist.

Es hat nicht an Versuchen gefehlt, durch Zusatz verschiedener Wachstumsfaktoren das Zellwachstum zu beschleunigen. So ist beispielsweise aus Gospodarovicz, D., J. biol. Chem. 250 (1975) 2515—2520 der »Fibroblast Growth Factor« (FGF) bekannt, der aus einem Rinder-Hypophysenextrakt gewonnen wird. Die bisher bekanntgewordenen Wachstumsfaktoren haben jedoch alle den Nachteil, daß sie eine mangelhafte Stabilität vor allem gegenüber Proteasen aufweisen, die den Wachstumsfaktor bereits in den zur Extraktion vorgesehenen Hypophysen angreifen.

Gebrauchsfertige Lösungen dieser Wachstumsfaktoren sind daher instabil. Sie werden als Lyophilisate in den Handel gebracht, die bei —20° C gelagert werden müssen. Sie müssen vor dem Gebrauch mit einem sterilen Lösungsmittel in Lösung gebracht werden. Die bisherige Praxis hat jedoch gezeigt, daß selbst die lyophilisierten Produkte bzw. die daraus regenerierten Lösungen große Aktivitätsunterschiede aufweisen. Es treten häufig Löslichkeitsprobleme auf, so daß Lösungen der bisher gebräuchlichen Wachstumsfaktoren nicht mit gleichbleibender Qualität hergestellt werden können. Die den bekannten Faktoren bisher zugeschriebenen positiven Wachstumseffekte haben sich in der Praxis nicht reproduzieren lassen. Die bisher im Handel erhältlichen Wachstumsfaktoren haben daher keine breite Anwendung bei der Züchtung der Fruchtwasserzellen und anderer Fibroblasten bzw. fibroblastenartiger Zellen gefunden.

Aus Chemical Abstracts, 95 (1981), 113272v sind Versuche bekannt, sekundäre, vorstimulierte Ratten-Fibroblasten zu vermehrter Synthese von Glucosaminoglycanen, DNS und Collagen anzuregen. Hierzu werden die Fibroblasten mit isolierten, lyophylisierten Makromolekülen aus Überständen von nichtstimulierten Makrophagen in Kontakt gebracht.

Aufgabe der vorliegenden Erfindung war es nun, ein Verfahren zur Züchtung von humanen Fibroblasten und fibroblastenartigen Zellen bereitzustellen, das weniger zeitaufwendig und deutlich zuverlässiger als die bisher bekannten Züchtungsmethoden ist. Gelöst wurde diese Aufgabe dadurch, daß man dem zur Züchtung verwendeten Kulturmedium einen Lymphokin-reichen Überstand von stimulierten Lymphozyten zusetzt.

Gegenstand der vorliegenden Erfindung ist daher ein Verfahren zur Züchtung von humanen Fibroblasten und fibroblastenartigen Zellen in einem üblichen Kulturmedium, dadurch gekennzeichnet, daß dem Kulturmedium ein Lymphokin-reicher Überstand von stimulierten Lymphozyten zugesetzt wird.

Als Lymphokine werden ganz allgemein Faktoren bezeichnet, die verschiedene biologische Aktivitäten von bestimmten Zellen, z. B. von T-Lymphozyten, an andere Zellen vermitteln. Man erhält Lymphokine, indem man bevorzugt Lymphozyten mit üblichen Stimulierungsmitteln, beispielsweise mit Mitogenen wie Concanavalin A (ConA), behandelt. Die Lymphokine reichern sich dabei in dem Überstand der Lymphozyten an und können von letzteren abzentrifugiert werden.

Ein im Sinne der vorliegenden Erfindung besonders vorteilhafter Lymphokin-reicher Überstand wird erhalten, wenn man vorinkubierte mononukleare Lymphozyten, die aus frischen Blutkonserven oder anderen Blutpräparaten gewonnen werden können, wie in J. Immunol. 125 (1980) S. 1823—1828 beschrieben, mit ConA stimuliert und den Überstand abzentrifugiert.

Der Lymphokin-reiche Überstand wird dem Kulturmedium in einer Konzentration von 2—20 Vol.-%, vorzugsweise 5—10 Vol.-% zugesetzt. Die erforderlichen Konzentrationen hängen im wesentlichen davon ab, wie stark die Lymphokin-Aktivität in dem Überstand angereichert werden konnte.

Der Zusatz des Lymphokin-reichen Überstandes zu dem Kulturmedium bewirkt, daß einmal die Anzahl der Zellkolonien in einem bestimmten Volumen in signifikanter Weise ansteigt und zum anderen, daß die Zahl der Zellen je Kolonie sowie die Anzahl der Zellen im Mitosestadium deutlich zunimmt. Dies hat beispielsweise bei der Züchtung der Fruchtwasserzellen zur Folge, daß nach dem erfindungsgemäßen Verfahren bereits nach 9—11 Tagen die Fruchtwasserzellen geerntet und analysiert werden können. Nach den bisher üblichen Methoden waren 2—3 Wochen erforderlich, um eine für die Analyse hinreichende Anzahl von Zellen bzw. Biomasse zu erhalten.

Es ist nicht erforderlich, für den erfindungsgemäßen Zweck den Lymphokin-reichen Überstand besonders zu reinigen. Der rohe Lymphozyten-Überstand kann direkt dem Kulturmedium zugesetzt werden. Es ist jedoch im allgemeinen zweckmäßig, den rohen Lymphozyten-Überstand weiter zu reinigen. Dies kann beispielsweise geschehen durch Adsorption an Blutzellen, insbesondere an Erythrozyten, oder auch mit Hilfe von Immunoadsorbentien, wobei vorzugsweise die Antikörper gegen das eingesetzte Stimulierungsmittel verwendet werden.

Lymphokin-reiche Überstände haben den weiteren Vorteil, daß sie in Lösung lagerstabil sind, mindestens 3 Monate bei 4—6° C. Sie können daher als direkt einsetzbare sterile Lösungen in den Handel gebracht werden. Löslichkeitsprobleme, wie sie beim Auflösen bisher üblicher Lyophilisate aufgetreten sind, entfallen.

Die Erfindung wird durch die folgenden Beispiele näher erläutert:

### Beispiel 1

### Mitoserate von Fruchtwasserzellkulturen

Fruchtwasserzellen enthaltende Proben werden in dem Kulturmedium RPMI 1640, dem 20 Vol.-% fetales Kälberserum und 10 Vol.-% eines durch Stimulierung mononuclearer Lymphozyten durch ConA nach J. Immunol. 125 (1980) S. 1823—1828 gewonnenen Lymphokin-reichen Lymphozyten-Überstandes zugesetzt worden ist (im folgenden ACGF ( = amniotic cell growth factor) enthaltender Überstand genannt), kultiviert (Proben A). Zwischen dem 9. und 13. Kulturtag werden die Zellen ausgezählt, die sich im Mitosestadium befinden.

Im Vergleich werden einmal Fruchtwasserzellen nur in dem Kulturmedium RPMI 1640 mit Zusatz von 20 Vol.-% fetalem Kälberserum kultiviert (Kontrolle). Ferner werden Fruchtwasserzellen im Kulturmedium RPMI 1640 gezüchtet, dem neben 20 Vol-.% fetales Kälberserum 10 Vol-.% eines Lymphozyten-Überstandes zugemischt worden sind, der in analoger Weise wie bei den Proben A gewonnen worden ist, jedoch mit dem Unterschied, daß das Stimulierungsmittel ConA nicht zu Beginn der Stimulierungsphase, sondern erst kurz vor der Aufarbeitung der Lymphozytenkultur zugegeben wird, d. h. es findet in diesem Falle keine Stimulierung der mononuclearen Lymphozyten durch ConA statt (Probe B).

In Tabelle 1 sind die Ergebnisse dieser Versuche zusammengefaßt.

### Tabelle 1

Mitoserate von Fruchtwasserzellen, kultiviert

A    in Kulturmedium RPMI mit 20 Vol-.% fetalem Kälberserum und 10 Vol-.% ACGF enthaltenden Überstand

B    in Kulturmedium RPMI 1640 mit 20 Vol-.% fetalem Kälberserum und 10 Vol-.% Überstand ohne ConA-Stimulierung

Kontrolle
in Kulturmedium RPMI 1640 mit 20 Vol-.% fetalem Kälberserum

n = Zahl der untersuchten Fruchtwasserproben; je Fruchtwasserprobe wurden 2 Kulturen untersucht.

|  | A | B | Kontrolle |
|---|---|---|---|
| n | 13 | 13 | 22 |
| Zellen im Mitosestadium | 264 | 95 | 107 |
| $(\bar{X} \pm S)$ | $\pm 72$ | $\pm 43$ | $\pm 38$ |

Die Ergebnisse in Tabelle 1 zeigen, daß in den Zellkulturen die mit Zusatz von ACGF enthaltendem Überstand kultiviert worden sind, die Zahl der Zellen im Mitosestadium deutlich erhöht ist im Vergleich zu den Zellkulturen, die nur nach der bisher üblichen Methode mit Kulturmedium RPMI 1640 mit 20 Vol.-% fetalem Kälberserum bzw. mit einem weiteren Zusatz eines nicht stimulierten Lymphozyten-Überstandes gezüchtet worden sind.

In Tabelle 2 sind die Zellkulturen aufgeführt, die bei den oben angegebenen Züchtungsmethoden nicht auswertbar waren.

Tabelle 2

Nicht auswertbare Fruchtwasserzellkulturen

|  | A | B | Kontrolle |
|---|---|---|---|
| n | 13 | 13 | 22 |
| nicht auswertbar | 1 | 5 | 8 |

Die in Tabelle 2 aufgeführten Daten zeigen, daß die Steigerung der Mitoserate durch ACGF enthaltende Überstände die Anzahl der nichtauswertbaren Kulturen zwischen dem 9. und 13. Kulturtag deutlich vermindert. Als nicht auswertbare Zellkultur wird eine Zellkultur angesehen, die weniger als 25 Zellen im Mitosestadium aufweist.

Beispiel 2

14 voneinander unabhängige Fruchtwasserproben wurden zwischen der 16. und 18. Schwangerschaftswoche durch Aminozenthese gewonnen. Von jeder Fruchtwasserprobe wurden in bekannter Weise 4 Kulturen nach folgendem Schema angelegt:

Kultur 1:
 Es wurde jeweils das Kulturmedium HAM's F10 der Fa. SEROMED mit 20 Vol-.% fetalem Kälberserum zugegeben.
Kultur 2:
 Wie Kultur 1, jedoch wurde vor der Zugabe des Mediums die Kulturschale mit fetalem Kälberserum vorbeschichtet.
Kultur 3:
 Es wurde kultiviert im Kulturmedium HAM's F10 mit 20 Vol-.% fetalem Kälberserum und 10 Vol-.% ACGF enthaltenden Überstand.
Kultur 4:
 Es wurde Kulturmedium HAM's F10 mit 20 Vol-.% fetalem Kälberserum und 2 µg ConA/ml zugegeben.

Die Kulturen wurden jeweils bei 37°C, 95% relativer Luftfeuchte und bei 3,6% Kohlendioxyd-Gehalt gezüchtet. Die erste Beurteilung erfolgte am 3.—4. Tag nach Anlegen der Kultur. Am 6.—7. Kulturtag erfolgte eine zweite Beurteilung. Beurteilt wurden jeweils die Zahl der Einzelzellen, die Zahl und die Größe der gewachsenen Zellhaufen (Klone), wie rasch und wie fest sich die Zellen an der Wand der Kulturschale festsetzen sowie wie rasch eine Diagnose erstellt werden konnte. Die 4 Kulturen je Fruchtwasserprobe wurden nach einem 6-Punkte-System beurteilt (bestes Ergebnis = 6 Punkte). Am 3.—4. Kulturtag ergab sich die folgende Reihenfolge:

 1. Kultur 3
 2. Kultur 4
 3. Kultur 2
 4. Kultur 1

Am 6.—7. Kulturtag ergab sich die gleiche Rangfolge.

Diese Rangfolge zeigt, daß die Kulturen mit dem ACGF-enthaltenden Überstand den übrigen Kulturen überlegen sind. Der Zusatz von ACGF-enthaltendem Überstand vermehrt die Zahl der Einzelzellen sowie die Zahl und Größe der Klone. Ferner bewirkt der ACGF-enthaltende Überstand, daß sich die Zellen rascher und fester an die Wand der Kulturschale anheften. Diese Vorteile ermöglichen eine schnellere Beurteilung der Fruchtwasserproben, wenn die Fruchtwasserzellen mit einem Medium gezüchtet werden, dem ACGF-enthaltender Überstand zugesetzt wird.

Beispiel 3

Drei verschiedene Fruchtwasserproben wurden jeweils in einer Zellkultur in bisher üblicher Weise mit dem Kulturmedium HAM's F10 mit 20 Vol-.% fetalem Kälberserum sowie in erfindungsgemäßer Weise mit dem Kulturmedium HAM's F10 mit 20 Vol-.% fetalem Kälberserum und 10 Vol-.% ACGF-enthaltendem Überstand untersucht. Die Kulturen ohne den ACGF-enthaltenden Überstand konnten, wie bisher üblich, erst nach 15—17 Tagen ausgewertet werden. Eine Auswertung der Kulturen mit dem ACGF-enthaltenden Überstand war bereits am 11. Kulturtag möglich. Durch Zusatz des ACGF-enthaltenden Überstandes bei der Züchtung der Fruchtwasserzellen konnte somit die Kulturdauer um 4—6 Tage verkürzt werden. Bei einer Kulturzeit von 15—17 Tagen war es bisher erforderlich, zweimal das Kulturmedium zu wechseln, bei Zusatz des ACGF-enthaltenden Überstandes ist nur noch ein Mediumwechsel erforderlich.

**Patentansprüche**

1. Verfahren zur Züchtung von humanen Fibroplasten und fibroplastenartigen Zellen in einem üblichen Kulturmedium, dem gegebenenfalls übliche Zusatzstoffe zugemischt werden, dadurch gekennzeichnet, daß dem Kulturmedium ein Lymphokin-reicher Überstand von stimulierten Lymphozyten zugesetzt wird.

2. Verfahren zur Züchtung von Fruchtwasserzellen in einem üblichen Kulturmedium, dem gegebenenfalls 5—20% fetales Kälberserum zugesetzt wird, dadurch gekennzeichnet, daß dem Kulturmedium ein Lymphokin-reicher Überstand von stimulierten Lymphozyten zugesetzt wird.

3. Verfahren gemäß einem der Ansprüche 1 oder 2, dadurch gekennzeichnet, daß der Lymphokin-reiche Überstand dem Kulturmedium in einer Konzentration von 2—20 Vol-.% zugesetzt wird.

4. Verfahren gemäß einem der Ansprüche 1—3, dadurch gekennzeichnet, daß der Lymphokin-reiche Überstand dem Kulturmedium in einer Konzentration von 5—10 Vol-.% zugesetzt wird.

5. Verfahren gemäß einem der Ansprüche 1—4, dadurch gekennzeichnet, daß der Lymphokin-reiche Überstand durch Stimulierung vorinkubierter mononuklearer Lymphozyten durch Mitogene erhältlich ist.

## Claims

1. Process for the culturing of human fibroblasts and fibroblast-like cells in a conventional culture medium, to which conventional additive materials are possibly admixed, characterised in that a lymphokine-rich supernatant from stimulated lymphocytes is added to the culture medium.

2. Process for the culturing of amniotic fluid cells in a conventional culture medium, to which 5 to 20% of foetal calf serum has optionally been added, characterised in that a lymphokine-rich supernatant from stimulated lymphocytes is added to the culture medium.

3. Process according to one of claims 1 or 2, characterised in that the lymphokine-rich supernatant is added to the culture medium in a concentration of 2 to 20 vol.-%.

4. Process according to one of claims 1—3, characterised in that the lymphokine-rich supernatant is added to the culture medium in a concentration of 5—10 vol.-%.

5. Process according to one of claims 1—4, characterised in that the lymphokine-rich supernatant is obtainable by stimulation of preincubated mononuclear lymphocytes by mitogens.

## Revendications

1. Procedé de culture de fibroblastes humains et de cellules du type des fibroblastes dans un milieu de culture habituel auquel on ajoute éventuellement des additifs habituels, caractérisé en ce que le milieu de culture est additionné d'une solution limpide de lymphocytes stimulés, riche en lymphocine.

2. Procédé de culture de cellules amniotiques dans un milieu de culture habituel, auquel on ajoute éventuellement 5 à 20% de sérum foetal de veau, caractérisé en ce que le milieu de culture est additionné d'une solution limpide de lymphocytes stimulés, riche en lymphocine.

3. Procédé selon l'une revendication 1 ou 2, caractérisé en ce que la solution limpide rich en lymphocine est ajoutée au milieu de culture en une concentration de 2 à 20% en volume.

4. Procédé selon l'une des revendications 1 à 3, caractérisé en ce que la solution limpide riche en lymphocine est ajoutée au milieu de culture en une concentration de 5 à 10% volume.

5. Procédé selon l'une des revendications 1 à 4, caractérisé en ce que la solution limpide riche en lymphocine est obtenue par stimulation de lymphocytes mononucléaires pré-incubés au moyen de mitogènes.